# EUROPEAN PATENT APPLICATION

(11) **EP 4 282 414 A2**
(43) Date of publication of application: **29.11.2023**
(21) Application number: 23194778.9
(22) Date of filing: 14.01.2020
(51) Int. Cl.: A61K 31/443

(54) **MODIFIED RELEASE TABLET FORMULATIONS CONTAINING PHOSPHODIESTERASE INHIBITORS**

(30) Priority: 15.01.2019 EP 19151782
(62) Divisional of application: 20700712.1
(71) Applicant: UNION therapeutics A/S, 2900 Hellerup (DK)
(72) Inventor: RASMUSSEN, Marianne, 2750 Ballerup (DK); HØY, Karin Green, 2750 Ballerup (DK); RAVN, Carsten, 2750 Ballerup (DK); PAJANDER, Jari, 2750 Ballerup (DK); BERTELSEN, Poul E, 2750 Ballerup (DK); PEDERSEN, Gitte Pommergaard, 2750 Ballerup (DK)
(74) Representative: HGF

(57) **Abstract**

The present invention relates to modified release tablet formulations for oral administration of phosphodiesterase inhibitors. The pharmaceutical formulations are useful in the treatment, prevention or alleviation of dermal diseases or conditions.

## Description

### FIELD OF THE INVENTION

The present invention relates to modified release tablet formulations for oral administration of phosphodiesterase inhibitors. The formulations are useful in the treatment, prevention or alleviation of dermal diseases or conditions.

### BACKGROUND OF THE INVENTION

The drug release and the chemical and physical characteristics of a drug substance, among other factors, can influence the degree of success of obtaining optimal therapy.

The use of modified release tablet formulations may control the release of the therapeutic agent and thus control drug absorption from gastrointestinal tract. However, it is often difficult to predict whether a particular modified release formulation will provide the desired release profile, and it has generally been found that it is necessary to carry out considerable experimentation to obtain modified release formulations having the desired effect.

Phosphodiesterases (PDE's) are enzymes that catalyse the hydrolysis of cyclic AMP and/or cyclic GMP in cells to 5-AMP and 5-GMP, respectively, and as such they are critical to cellular regulation of cAMP or cGMP levels. Phosphodiesterase 4 (PDE4), is selective for cAMP. PDE4 is the most important modulator of cAMP expressed in immune and inflammatory cells such as neutrophils, macrophages and T-lymphocytes. As cAMP is a key second messenger in the modulation of inflammatory responses, PDE4 has been found to regulate inflammatory responses of inflammatory cells by modulating proinflammatory cytokines such as TNF-o, IL-2, IFN-γ, GM-CSF and LTB4. Inhibition of PDE4 has therefore become an attractive target for the therapy of inflammatory diseases such as asthma, chronic obstructive pulmonary disease, rheumatoid arthritis, atopic dermatitis, psoriasis, inflammatory bowel disease etc. (M.D. Houslay et al., Drug Discovery Today 10 (22), 2005, pp. 1503-1519).

PDE4 inhibitors have been associated with adverse effects found when administered to patients, primarily gastrointestinal side effects such as nausea, diarrhoea, and emesis.

In a clinical trial where patients were dosed with immediate release tablets containing the PDE4 inhibitor of formula (I) of the present invention, the results demonstrated that the PDE4 inhibitor was effective in patients with moderate to severe psoriasis vulgaris, but the extent of gastrointestinal related adverse events experienced were unacceptable.

Thus, there exists a need for a pharmaceutical formulation for oral administration of a PDE inhibitor, which could maintain systemic exposure and reduce gastrointestinal adverse events.

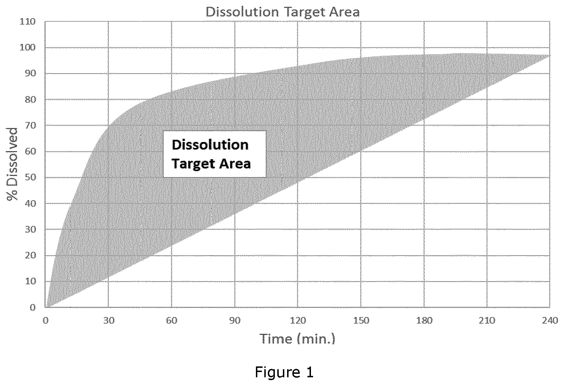

It has been found that beneficial effects with respect to improved tolerability towards gastrointestinal adverse events and maintained systemic exposure have been achieved by formulating a PDE4 inhibitor in a modified release tablet formulation, wherein the in-vitro release is fast in comparison to a typically modified release profile but not yet as fast as for an immediate release tablet where major tolerability issues were seen. The Dissolution target area in Figure 1 illustrates the optimal area.

### SUMMARY OF THE INVENTION

One object of the present invention is to provide modified release tablet formulations for oral administration of a PDE inhibitor, which formulations are useful in the treatment, prevention or alleviation of dermal diseases or conditions.

Another object of the present invention is to provide a modified release tablet formulation for oral administration of a PDE inhibitor, which shows beneficial effects with respect to maintained systemic exposure and improved tolerability towards reduced gastrointestinal adverse events.

It is hypothesised that an important driver for the gastrointestinal adverse events could be related to the local concentration of the PDE4 inhibitor in the gut and that higher local concentration of the PDE4 inhibitor could cause increased level of gastrointestinal adverse events.

Upon entering the gastrointestinal tract, if the PDE4 inhibitor could slowly be released and dissolved, thus a high local concentration of PDE4 inhibitor could be prevented in the intestinal fluids.

It has been found that when the PDE4 inhibitor was given orally a very narrow absorption window existed in the upper part of the gastrointestinal tract. Therefore, to maintain the systemic exposure the release and dissolution of the drug substance from the modified release tablet formulation must take place in the upper part of the gastrointestinal tract.

In one aspect, the present invention relates to a modified release tablet formulation for oral administration of a PDE inhibitor, comprising:
(i) a PDE inhibitor;
(ii) one or more of a pharmaceutically acceptable hydrophilic matrix former;
(iii) one or more pharmaceutically acceptable excipients selected from the group consisting fillers, glidants and lubricants; and
(iv) optionally a pharmaceutically acceptable coating system.

In another aspect, the present invention relates to a modified release tablet formulation wherein the PDE inhibitor is a PDE4 inhibitor.

In another aspect, the present invention relates to a modified release tablet formulation for oral administration wherein the PDE4 inhibitor is a compound of formula (I) 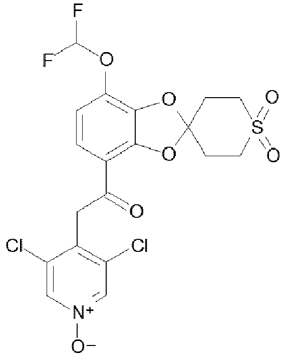 or a pharmaceutically acceptable salt, or polymorphic forms thereof.

The compound of formula (I), 2-(3,5-Dichloro-1-oxido-pyridine-4-yl)-1-(7-difluoro-methoxy-2',3',5',6'-tetrahydro-spiro[1,3-benzodioxole-2, 4'-(4H)-thiopyran-1',1'-dioxide]-4-yl)ethanone, hereafter named Compound A, was disclosed in WO 2011/160632, relating to benzodioxole and benzodioxepene heterocyclic compounds useful as PDE4 inhibitors for use in the treatment, prevention or alleviation of a variety of diseases, such as dermal diseases or conditions, such as proliferative and inflammatory skin disorders, dermatitis, psoriasis, psoriasis vulgaris, atopic dermatitis, seborrheic dermatitis, contact dermatitis, cancer, epidermal inflammation, alopecia, alopecia areata, skin atrophy, steroid induced skin atrophy, skin ageing, photo skin ageing, acne, urticaria, pruritis, and eczema.

The compound A should be understood to include any pharmaceutically acceptable form and salts of the compound. The compound A may be present in a crystalline or amorphous form. Compound A is considered as being a poorly soluble compound. The compound A and salts thereof, and methods for synthesizing the compound, are disclosed in WO 2011/160632, WO 2015/197534, WO 2017/103058, and WO 2018/234299.

In another aspect, the present invention relates to a modified release tablet formulation for oral administration of PDE4 inhibitors in the treatment, prevention or alleviation of dermal diseases or conditions selected from the group consisting of proliferative and inflammatory skin disorders, dermatitis, psoriasis, psoriasis vulgaris (plaque-type psoriasis), atopic dermatitis, seborrheic dermatitis, contact dermatitis, cancer, epidermal inflammation, alopecia, alopecia areata, skin atrophy, steroid induced skin atrophy, skin ageing, photo skin ageing, acne, urticaria, pruritis, and eczema.

In another aspect, the present invention relates to a modified release tablet formulation for oral administration of PDE4 inhibitors in the treatment, prevention or alleviation of psoriasis vulgaris.

In another aspect, the present invention relates to a modified release tablet formulation for oral administration of PDE4 inhibitors in the treatment, prevention or alleviation of moderate to severe psoriasis vulgaris.

In another aspect, the present invention relates to a modified release tablet formulation wherein the in-vitro release is fast in comparison to a typically modified release profile but not yet as fast as for an immediate release tablet, as indicated in the background section of the present application.

The rate of dissolution will be determined by several factors e.g. the type and quantity of hydrophilic matrix former, excipients (fillers and coating) and the particle size of the drug substance.

The details of one or more embodiments of the inventions are set forth in the description below. Other features, objects and advantages of the inventions will be apparent from the description and claims.

### BRIEF DESCRIPTION OF THE FIGURES

Figure 1. A chart illustrating the dissolution target area; dissolution method: Paddle 75 rpm, 900 ml 0.1N HCl +0.5%SDS, 37°C, HPLC detection.
   The curves presented in figure 2 and 3 describe the release of compound A from the formulations F1 and F2, respectively, when tested by the dissolution method parameters described on page 11.
Figure 2. Dissolution profile for F1.
Figure 3. Dissolution profile for F2.
Figure 4. A chart illustrating the presence of gastrointestinal adverse events in subject, with onset when dosed twice daily by 10mg, 20mg or 30mg of the PDE4 inhibitor of formula (I) of the present invention in Modified Release tablets; each dot represent one gastrointestinal related adverse event and the bar the duration of the adverse event.
Figure 5. A chart illustrating the presence of gastrointestinal adverse events in subject, with onset when dosed twice daily by 10mg, 20mg or 30mg of the PDE4 inhibitor of formula (I) of the present invention in Immediate Release tablets; each dot represent one gastrointestinal related adverse event and the bar the duration of the adverse event.

### DETAILED DISCLOSURE OF THE INVENTION

### Definitions

As used throughout the present specification and appended claims, the following terms have the indicated meaning.

The phrase "phosphodiesterase" as used herein refers to one or more of the phosphodiesterases (PDEs), PDE4, PDE7 and PDE8 being selective for cAMP. PDE4 is the most important modulator of cAMP.

The phrase "PDE inhibitor" as used herein may be any substances which inhibit PDE. The PDE inhibitor is preferably a human PDE inhibitor. The PDE inhibitor is preferably a PDE4 inhibitor. For example, the PDE4 inhibitor could be Compound A, or a pharmaceutically acceptable salt, or polymorphic forms thereof, preferably Compound A, and more preferably the polymorphic form E of Compound A.

The term "treatment" as used herein includes amelioration of a symptom, prevention of an aggravation, maintenance of a remission, prevention of an exacerbation, and prevention of a recurrence. The term "prevention" refers to suppressing occurrence of a symptom.

The term "treatment" may also include the delaying of the progression of the disease, disorder or condition, the amelioration, alleviation or relief of symptoms and complications, and/or cure or elimination of the disease, disorder or condition.

The terms "disease", "disorder" and "condition" as used herein are used interchangeably to specify a state of a patient which is not the normal physiological state of a human being.

The term "hydrophilic matrix former" as used herein includes hydroxypropyl methylcellulose (HPMC) or hydroxypropylcellulose (HPC). For example, the hydrophilic matrix former could be hydroxypropyl methylcellulose, or mixtures thereof.

The term "filler" as used herein includes lactose, for example lactose monohydrate, lactose hydrous or anhydrous, microcrystalline cellulose, mannitol, isomalt, ect. For example, the filler could be lactose monohydrate, microcrystalline cellulose, or a mixture thereof.

The term "filler" as used herein may also function as a binder.

The term "glidant" as used herein includes colloidal silicon dioxide, talc, ect. For example, the glidant could be colloidal silicon dioxide.

The term "lubricant" as used herein includes magnesium stearate, sodium stearyl fumarate, talc, ect. For example, the lubricant could be magnesium stearate.

The term "coating system", as used herein includes HPMC-based coating systems, PVA-based coating systems (polyvinyl alcohol), PVA-PEG based coating systems (polyethylene glycol) or ethylcellulose based functional barrier membrane coating systems.. For example, the coating system could be the PVA-based coating system.

The term "about" is used herein to mean approximately, in the region of, roughly, or around.

### Embodiments

Embodiments of the modified release tablet formulation may include one or more of the following features.

In one embodiment of the modified release tablet formulation the PDE inhibitor is a PDE4 inhibitor.

In another embodiment the PDE4 inhibitor is evenly distributed.

In another embodiment the PDE4 inhibitor is micronized.

In another embodiment the PDE4 inhibitor is crystalline and micronized.

In another embodiment of the modified release tablet formulation, the PDE4 inhibitor is compound A.

In another embodiment the PDE4 inhibitor is compound A, and preferably the polymorphic form E of Compound A.

In another embodiment compound A is micronized. In another embodiment the polymorphic Form E of compound A is micronized.

In another embodiment compound A is evenly distributed. In another embodiment the polymorphic Form E of compound A is is evenly distributed.

In another embodiment, compound A is crystalline and micronized.

In another embodiment, the polymorphic Form E of compound A is crystalline and micronized.

In another embodiment the modified release tablet formulation could contain a hydrophilic matrix former, or mixtures thereof. For example, the hydrophilic matrix former could be hydroxypropyl methylcellulose (HPMC), hydroxypropylcellulose (HPC), or mixtures thereof. For example, the hydrophilic matrix former could be hydroxypropyl methylcellulose, and mixtures thereof.

The hydrophilic matrix former could be present at various concentrations and combinations from about 10 %w/w to about 30 %w/w HPMC, for example from about 15 %w/w to about 25 %w/w HPMC, and specifically 17,5 %w/w HMPC.

In another embodiment the modified release tablet formulation could comprise one or more fillers/binders selected from lactose monohydrate, lactose hydrous or anhydrous, microcrystalline cellulose, and mixtures thereof. For example, the filler could be lactose monohydrate.

The filler could be present at various concentrations from about 30 %w/w to about 78 %w/w of lactose monohydrate and from about 0 to about 40 %w/w of microcrystalline cellulose. For example, the filler could be lactose monohydrate in a 71 %w/w.

In another embodiment the modified release tablet formulation could comprise one or more glidants. For example, the glidant could be colloidal silicon dioxide.

The glidant could be present at various concentrations from about 0.1 %w/w to about 2 %w/w of colloidal silicon dioxide, for example from about 0.2 %w/w to about 1 %w/w, and specifically 0.5%w/w.

In another embodiment the modified release tablet formulation could comprise one or more lubricants. For example, the lubricant could be magnesium stearate.

The lubricant could be present at various concentrations from about 0.1 %w/w to about 2 %w/w of magnesium stearate, for example from about 0.5 %w/w to about 1.5%w/w, and specifically 1.0 %w/w.

In another embodiment the modified release tablet formulation could comprise a film coating of the tablet cores.

In another embodiment the coating system could be a PVA-based coating system. For example, the coating system could be Opadry ^{®} II. For example the coating system could be present in an amount from about 3% to about 5 % weight gain of the tablet formulation, and specifically a 4 % weight gain.

In another embodiment of the present invention, the particle size distribution of the PDE inhibitor could have a D₅₀ ≤ 25 µm, for example D₅₀ ≤ 20 µm, D₅₀ ≤ 10 µm, D₅₀ ≤ 5 µm, or D₅₀ ≤ 3 µm.

In another aspect, the amount of the PDE inhibitor may range from about 5 mg to about 60 mg. The amount of PDE inhibitor may for example range from 10 mg to 50 mg, from 20 mg to 45 mg, and from 30 mg to 40 mg.

In another aspect, the present invention relates to a method of treating psoriasis vulgaris. The method includes administering to a patient in need thereof, a modified release tablet formulation containing a PDE inhibitor.

In another aspect, the present invention relates to the method of treating psoriasis vulgaris in a patient in need thereof, which method includes administering a modified release tablet formulation containing compound A in a concentration of 10 mg.

In another aspect, the present invention relates to the method of treating psoriasis vulgaris in a patient in need thereof, which method includes administering a modified release tablet formulation containing compound A in a concentration of 30 mg.

In another aspect, the present invention relates to the method of treating psoriasis vulgaris in a patient in need thereof, which method includes administering a modified release tablet formulation containing compound A in a concentration of 30 mg, twice daily.

In another aspect of the present invention, there is provided a process for the preparation of the modified release tablet formulation wherein the manufacturing process consisted of blending and sieving steps of the drug substance and excipients followed by direct compression, or roller compaction followed by compression, and finally optionally coating.

In another aspect, the present invention could relate to a granulated blend formulation comprising the PDE inhibitor of the present invention; one or more of a pharmaceutically acceptable hydrophilic matrix former; one or more pharmaceutically acceptable excipients selected from the group consisting fillers, binders, glidants and lubricants; and a hard capsule shell material.

The hydrophilic matrix former could be hydroxypropyl methylcellulose (HPMC), hydroxypropylcellulose (HPC), or mixtures thereof. The hydrophilic matrix former could be present at various concentrations and combinations from about 10 %w/w to about 20 %w/w HPMC

The fillers/binders could be selected from lactose monohydrate, lactose hydrous or microcrystalline cellulose, and mixtures thereof. The fillers/binders could be present at various concentrations from about 20 %w/w to about 75 %w/w of lactose monohydrate and from 0 to about 50%w/w of microcrystalline cellulose.

The glidant could be colloidal silicon dioxide, which could be present at various concentrations from about 0.1 %w/w to about 2 %w/w.

The lubricant could be magnesium stearate, which could be present at various concentrations from about 0.1 %w/w to about 2 %w/w.

The blend formulation could be dispensed in a hard capsule. Capsule shell material for hard capsules could be made of several materials such as gelatin (pig, bovine, fish etc), hydroxypropyl methylcellulose (HPMC), polyvinyl alcohol, starch and pullulan could be applied.

A manufacturing process for the granulated blend formulation could consist of blending and sieving steps of the drug substance and excipients followed by granulation, e.g. roller compaction, and encapsulation.

### EXAMPLES

### EXAMPLE 1

### Modified release tablet formulations

**Table 1. Modified release tablet formulations (F) for 10 mg and 30 mg dose strengths (in percentage w/w).**

| Ingredient | F 1 (10mg) | F 2 (30mg) | F 3 (30mg) | F 4 (30mg) | F 5 (30g) | F 6 (30mg) | F7 (10mg) |
|---|---|---|---|---|---|---|---|
| | Direct compression | Direct compression | Direct compression | Direct compression | Direct compression | Roller compac tion | Roller compac tion |
| **Drug substance** | | | | | | | |
| Compound A (micronized) | 3.3 % | 10.0 % | 10.0 % | 10.0 % | 10.0 % | 10.0 % | 3.3% |

| **Excipients** | | | | | | | |
|---|---|---|---|---|---|---|---|
| Lactose monohydrate | 77.7 % | 71.0 % | 73.5 % | 36.7 % | 50.0 % | 49.5 % | 49.5 % |
| Microcrystallin e cellulose | - | - | - | 36.7 % | 14.0 % | 14.0 % | 20.5 % |
| Hydroxypropyl methylcellulose (Methocel^{®} E50 LV | - | - | - | - | 25.0 % | 25.0 % | 20.0 % |
| Hydroxypropyl methylcellulose (Methocel^{®} K100 LV) | 17.5 % | 17.5 % | 15.0 % | 15.0 % | - | - | 5.0% |
| Colloidal silicon dioxide | 0.5 % | 0.5 % | 0.5 % | 0.5 % | - | 0.5 % | 0.5 % |
| Magnesium Stearate | 1.0 % | 1.0 % | 1.0 % | 1.0 % | 1.0 % | 1.0 % | 1.0 % |

| **Film coating** | | | | | | | |
|---|---|---|---|---|---|---|---|
| Coating system (% weight gain) | 4 % Opadry ^{®} II | 4 % Opadry ^{®} II | Not coated | 4 % Opadry ^{®}II | Not coated | 4 % Surelea se/Opa dry | Not coated |

### EXAMPLE 2

### Dissolution profiles for formulations F1 and F2

The release of compound A from the modified release tablet was investigated by in-vitro dissolution method, see below.

### Dissolution method:

Dissolution apparatus: USP/Ph.Eur. app.II (paddles)
75 rpm, 900 ml 0.1N HCl +0.5%SDS, 37°C, HPLC detection.

### Modified release formulation F1

Dissolution: % of declared amount

| time (min) | mean | SD |
|---|---|---|
| 6 | 7 | 1.0 |
| 12 | 15 | 1.1 |
| 20 | 22 | 1.3 |
| 30 | 28 | 1.7 |
| 45 | 36 | 2.2 |
| 60 | 42 | 2.2 |
| 90 | 54 | 2.6 |
| 120 | 64 | 3.0 |
| 180 | 81 | 2.6 |

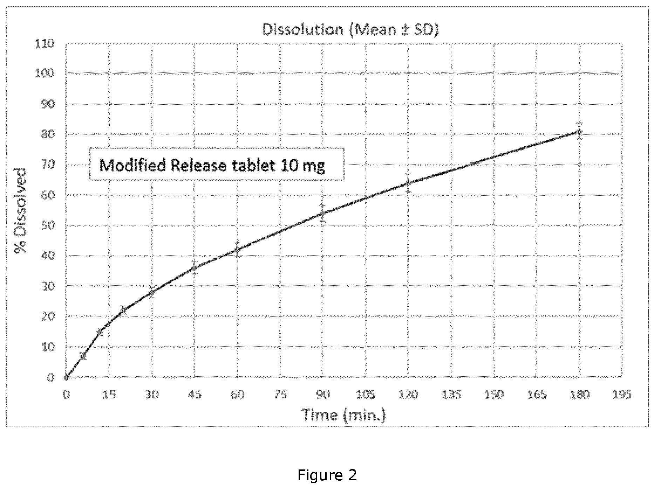

Figure 2 shows the dissolution profile for F1

### Modified release formulation F2

Dissolution: % of declared amount

| time (min) | mean | SD |
|---|---|---|
| 6 | 7 | 1.0 |
| 12 | 14 | 0.7 |
| 20 | 23 | 1.0 |
| 30 | 31 | 1,7 |
| 45 | 39 | 2.7 |
| 60 | 46 | 3.6 |
| 90 | 56 | 4.3 |
| 120 | 66 | 4.1 |
| 180 | 82 | 5.6 |

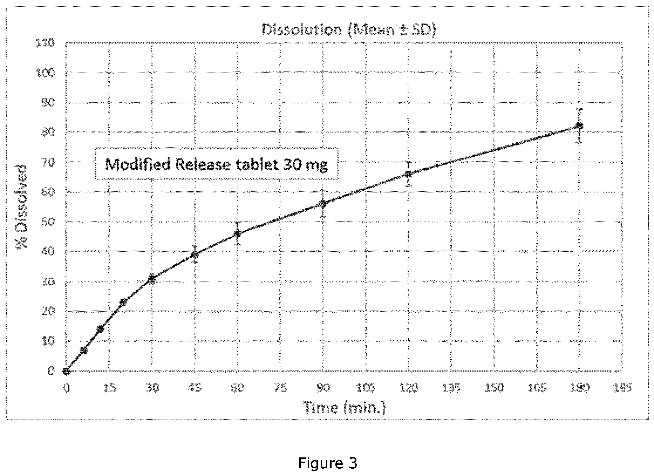

Figure 3 shows the dissolution profile for F2

### EXAMPLE 3

In an oral dose clinical trial, healthy subjects were dosed with the PDE4 inhibitor of formula (I) in the Modified Release tablet formulation of the present invention. The subjects received twice daily doses of: 10 mg of the compound of formula (I) on Days 1-2, 20 mg of the compound of formula (I) on Days 3-4, 30 mg of the compound of formula (I) on Days 5-6, 40 mg of the compound of formula (I) on Days 7-8, 50 mg of the compound of formula (I) on Days 9-10, and 60 mg of the compound of formula (I) on Days 11-17.

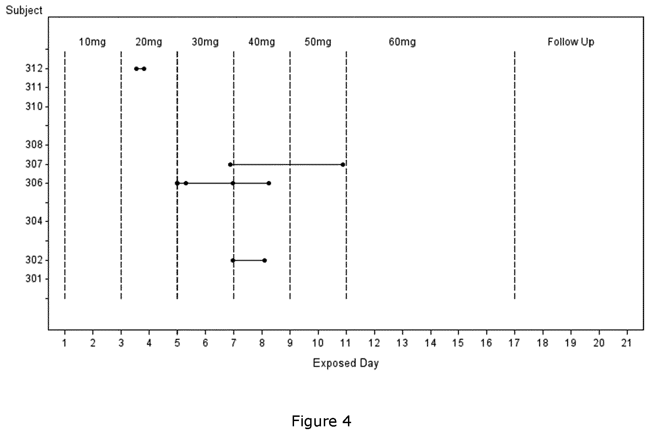

Gastrointestinal adverse advents in the subjects were collected in connection with onset when dosed by 10mg, 20mg or 30mg of the PDE4 inhibitor of formula (I), as shown in Figure 4.

Results from this Modified Release tablet formulation study (Figure 4) was compared with the results from a previous completed comparable oral dose clinical trial, where healthy subjects were dosed with the PDE4 inhibitor of formula (I) of the present invention in an Immediate Release tablet formulation.

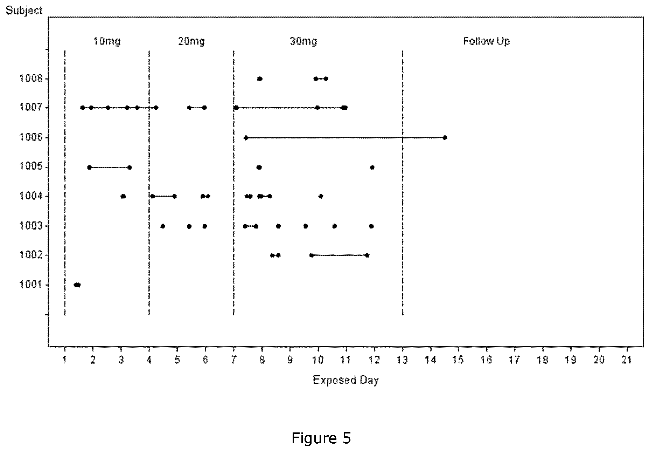

In the Immediate Release tablet formulation study, subjects received twice daily doses of: 10 mg of the PDE4 inhibitor of formula (I) of the present invention on Days 1-3, 20 mg of the compound of formula (I) on Days 4-6, and 30 mg of the compound of formula (I) on Days 7-13. Gastrointestinal adverse advents in the subjects were collected in connection with onset when dosed by 10mg, 20mg or 30mg of the PDE4 inhibitor of formula (I). The results which are shown in Figure 5, demonstrates that the Immediate Release tablet formulation was poorly tolerated.

When comparing the results shown in Figure 4 (presence of gastrointestinal related adverse events in subjects when dosed with Modified Release tablets containing the PDE4 inhibitor of formula (I)) with the results shown in Figure 5 (presence of gastrointestinal related adverse events in subjects when dosed with Immediate Release tablets containing the PDE4 inhibitor of formula (I)), the Modified Release tablet formulation study shows that the total number of gastrointestinal adverse events was clinically significantly lower when compared to the immediate release tablets.

The present application and invention further includes the subject matter of the following numbered clauses:
1. A modified release tablet formulation comprising:
   (i) a PDE inhibitor;
   (ii) one or more of a pharmaceutically acceptable hydrophilic matrix former;
   (iii) one or more pharmaceutically acceptable excipients selected from the group consisting fillers, glidants and lubricants; and
   (iv) optionally a pharmaceutically acceptable coating system.
2. The modified release tablet formulation according to clause 1 wherein the hydrophilic matrix former comprises one or more of hydroxypropylmethylcellulose, or mixtures thereof.
3. The modified release tablet formulation according to clause 2 wherein the hydroxypropylmethylcellulose is hypromellose 2910, hypromellose 2208, or mixtures thereof.
4. The modified release tablet formulation according to any one of clauses 1-3, wherein the hydrophilic matrix former is present in a concentration from about 10 %w/w to about 30 %w/w hydroxypropylmethylcellulose, e.g. from 15 %w/w to about 25 %w/w, and specifically 17,5%w/w.
5. The modified release tablet formulation according to any one of clauses 1-4 wherein two of the pharmaceutically acceptable excipients are fillers, selected from lactose monohydrate and microcrystalline cellulose.
6. The modified release tablet formulation according to clause 5, wherein the fillers are present in a concentration from about 30 % to about 78%w/w of lactose monohydrate and from 0 to about 40 %w/w of microcrystalline cellulose.
7. The modified release tablet formulation according to clause 5 or 6, wherein the filler is present in a concentration of about 71 %w/w lactose monohydrate.
8. The modified release tablet formulation according to any one of clauses 1-7 wherein one of the pharmaceutically acceptable excipients is a glidant, which is colloidal silicon dioxide.
9. The modified release tablet formulation according to clause 8, wherein the glidant is present in a concentration from about 0.1 %w/w to about 2 %w/w of colloidal silicon dioxide, for example from about 0.2 %w/w to about 1 %w/w, and specifically 0.5 %w/w.
10. The modified release tablet formulation according to any one of clauses 1-9 wherein one of the pharmaceutically acceptable excipients is a lubricant, which is magnesium stearate.
11. The modified release tablet formulation according to clause 9 wherein the lubricant is present in a concentration from about 0.1 %w/w to about 2 %w/w of magnesium stearate, for example from about 0.5 %w/w to about 1.5 %w/w, and specifically 1.0 %w/w.
12. The modified release tablet formulation according to any one of clauses 1-11 wherein the coating system is a PVA-based coating system.
13. The modified release tablet formulation according to any one of clauses 1-12 wherein the phosphodiesterase inhibitor is a PDE4 inhibitor.
14. The modified release tablet formulations according to clause 13 wherein the PDE4 inhibitor is a compound of formula (I) 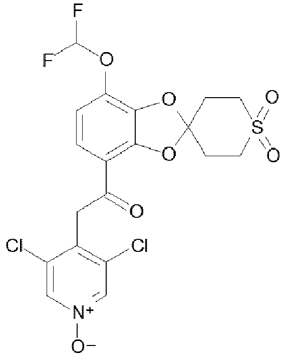 or a pharmaceutically acceptable salt, or polymorphic forms thereof.
15. The modified release tablet formulation according to clause 14 wherein the compound is 2-(3,5-Dichloro-1-oxido-pyridine-4-yl)-1-(7-difluoromethoxy-2',3',5',6'-tetrahydro-spiro[1,3-benzodioxole-2,4'-(4H)-thiopyran-1',1 '-dioxide]-4-yl)ethenone, polymorphic form E.
16. The modified release tablet formulation according to any one of clauses 1-15 wherein the compound is in micronized form.
17. The modified release tablet formulation according to any one of clauses 1-16 wherein the compound has a particle size distribution with D₅₀ ≤ 5 µm.
18. The modified release tablet formulation according to any one of clauses 1-16 wherein the formulation consists of about 3.3 %w/w micronized 2-(3,5-Dichloro-1-oxido-pyridine-4-yl)-1-(7-difluoromethoxy-2',3',5',6'-tetrahydro-spiro[1,3-benzodioxole-2, 4'-(4H)-thiopyran-1',1'-dioxide]-4-yl)ethenone, about 17.5 %w/w hypromellose, about 77.7 %w/w lactose monohydrate, about 0.5 %w/w colloidal silicon dioxide, about 1.0 %w/w magnesium stearate; and optionally a PVA-based coating system.
19. The modified release tablet formulation according to any one of clauses 1-16 wherein the formulation consists of about 10.0 %w/w micronized 2-(3,5-Dichloro-1-oxido-pyridine-4-yl)-1-(7-difluoromethoxy-2',3',5',6'-tetrahydro-spiro[1,3-benzodioxole-2, 4'-(4H)-thiopyran-1',1'-dioxide]-4-yl)ethenone, about 17.5 %w/w hypromellose, about 71.0 %w/w lactose monohydrate, about 0.5 %w/w colloidal silicon dioxide, about 1.0 %w/w magnesium stearate; and optionally a PVA-based coating system.

## Claims

1. A modified release tablet formulation for oral administration comprising a compound of the formula (I): 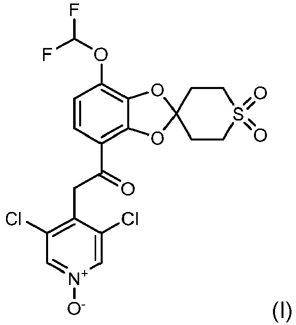 wherein the tablet formulation has a dissolution profile within the Dissolution Target Area shown in Figure 1 when measured in 900 ml 0.1N HCl +0.5 % SDS with a paddle speed of 75 rpm at a temperature of 37°C.

2. The modified release tablet formulation according to claim 1 comprising a hydrophilic matrix former, or mixtures thereof.

3. The modified release tablet formulation according to claim 1 or claim 2 comprising
(i) the compound of formula (I);
(ii) one or more of a pharmaceutically acceptable hydrophilic matrix former; and
(iii) one or more pharmaceutically acceptable excipients selected from the group consisting of fillers, glidants and lubricants;
wherein the modified release tablet formulation optionally further comprises a pharmaceutically acceptable coating system.

4. The modified release tablet formulation according to claim 2 or claim 3, wherein the hydrophilic matrix former comprises hydroxypropyl methylcellulose (HPMC), hydroxypropylcellulose (HPC), or mixtures thereof.

5. The modified release tablet formulation according to any one of claims 2 to 4, wherein the hydrophilic matrix former is present in an amount of from about 10 %w/w to about 30 %w/w HPMC.

6. The modified release tablet formulation according to any one of claims 1 to 5, comprising one or more fillers or binders selected from lactose monohydrate, lactose hydrous or anhydrous, microcrystalline cellulose, and mixtures thereof, for example wherein the filler is lactose monohydrate.

7. The modified release tablet formulation according to any one of claims 1 to 6, wherein the tablet further comprises a film coating.

8. The modified release tablet formulation according to any one of claims 1 to 6, wherein the modified release tablet formulation further comprises a coating system,
optionally wherein the coating system comprises coating system selected from: a hydroxypropyl methylcellulose-based coating system, a polyvinyl alcohol-based coating system, a polyvinyl alcohol-polyethylene glycol-based coating system and an ethylcellulose-based functional barrier membrane coating system.

9. The modified release tablet formulation according to claim 8, wherein the coating system is a PVA-based coating system.

10. The modified release tablet formulation according to claim 8 or claim 9, wherein the coating is present in an amount of from about 3% to about 5 % weight gain of the tablet formulation, preferably a 4 % weight gain.

11. The modified release tablet formulation according to any one of claims 1 to 10, wherein the article size distribution of compound of formula (I) has a have a D₅₀ ≤ 25 µm, for example D₅₀ ≤ 20 µm, D₅₀ ≤ 10 µm, D₅₀ ≤ 5 µm, or D₅₀ ≤ 3 µm.

12. The modified release tablet formulation according to any one of claims 1 to 11, wherein the compound of formula (I) is present in a crystalline or amorphous form,
preferably wherein the compound of formula (I) is present in a crystalline and micronized form.

13. The modified release tablet formulation according to any one of claims 1 to 12, wherein the compound of formula (I) is present in an amount from about 5 mg to about 60 mg;
optionally wherein:
(i) the compound of formula (I) is present in an amount of 10 mg;
(ii) the compound of formula (I) is present in an amount of 20 mg;
(iii) the compound of formula (I) is present in an amount of 30 mg; or
(iv) the compound of formula (I) is present in an amount of 40 mg.

14. The modified release tablet formulation according to any one of claims 1 to 13, for use in the treatment, prevention or alleviation of a dermal disease or condition selected from the group consisting of a proliferative and inflammatory skin disorder, dermatitis, psoriasis, psoriasis vulgaris (plaque-type psoriasis), atopic dermatitis, seborrheic dermatitis, contact dermatitis, cancer, epidermal inflammation, alopecia, alopecia areata, skin atrophy, steroid induced skin atrophy, skin ageing, photo skin ageing, acne, urticaria, pruritis, and eczema;
wherein the modified release tablet formulation is administered orally.

15. The modified release tablet formulation, according to any one of claims 1 to 13, for use in the treatment of an inflammatory skin disorder, wherein the modified release tablet formulation is administered orally;
optionally wherein the modified release tablet formulation is for use in the treatment of psoriasis or atopic dermatitis.
